# EUROPEAN PATENT SPECIFICATION

(11) **EP 1 037 574 B1**
(45) Date of publication and mention of the grant of the patent: **14.06.2006**
(21) Application number: 98958406.5
(22) Date of filing: 08.12.1998
(51) Int. Cl.: A61F 2/20, A61M 16/04, A61M 16/10

(54) **SPEECH VALVE ASSEMBLY**
SPRECHVENTILANORDNUNG
ENSEMBLE SOUPAPES VOCAL

(30) Priority: 08.12.1997 NL 1007736
(43) Date of publication of application: 27.09.2000
(73) Proprietor: TRACOE medical GmbH, 60528 Frankfurt am Main (DE)
(72) Inventor: Schouwenburg, Paul Ferdinand, 2111 GH Aerdenhout (NL)
(74) Representative: Lieke, Winfried
(86) International application number: PCT/NL1998/000703
(87) International publication number: WO 1999/029268

(56) References cited:
- WO-A-94/01158
- WO-A-94/01199
- WO-A-95/17138
- FR-A- 2 683 150

## Description

The invention relates to a valve intended for fitting on the tracheal stoma of a laryngectomee. In the case of such a patient a voice prosthesis is placed in a puncture channel (fistula) between trachea and oesophagus, by means of which compressed air can be guided out of the trachea through the oesophagus and into the oral cavity. This gives the patient the opportunity of speech.

For speech, the air therefore must not be discharged through the tracheal stoma, but through the voice prosthesis. In order to achieve this, the patient can shut off the tracheal stoma using his finger, but it is more comfortable to provide the above mentioned speech valve for that purpose. Under the influence of a slightly increased pressure or outflow velocity, the patient can make the valve close, so that the desired diversion through the speech prosthesis is obtained.

A valve for a tracheal stoma is known per se from US-A-4582058. This valve comprises a tube with a passage which can be placed over the tracheal stoma. In order to prevent problems during inhalation of ambient air, a filter also has to be fitted on the valve. During exhalation said filter is heated and moistened, in such a way that the subsequently inhaled ambient air is preheated and brought to a higher degree of humidity. The ambient air thus pretreated conditions the air and substantially limits chronic respiratory tract complaint. Unconditioned (outside) air is too cold and too dry.

The known valve has a number of disadvantages. First of all, its construction is quite complex, and therefore expensive. Furthermore, this valve produces background noise when it is closing, and it quickly becomes soiled with mucous as a result of, for example, coughing. Moreover, the valve can shoot off during a severe coughing fit, or the skin seal can also be tom off. In combination with a filter, the total dimensions become quite large.

In prior art document WO 94/01199 a speech valve is disclosed comprising a valve seat and a spring biased valve plate. The valve plate is pressed against the valve seat under the influence of an enhanced pressure or outflow velocity of air. The forces needed to close the valve are predetermined by the spring's bias and cannot be adjusted to the patient's needs.

The object of the invention is to provide a solution to the problems raised by the prior art. That is achieved by means of a speech valve assembly for connection to the tracheal stoma of a laryngectomee, comprising a housing with a passage extending between a proximal end adapted to be positioned near the tracheal stoma and a free end situated opposite a filter means situated near the proximal end, and a valve situated on the side of the filter means facing the free end, said valve comprising a valve body and a valve seat which can be made to interact with each other against the initial tension, wherein said valve seat points towards the proximal end, such that the valve body and the valve seat can be made to interact with each other under the influence of an air flow from the proximal to the free end through the passage of the housing, wherein the valve seat is adjustable in the direction of passage through the housing.

The disadvantage of this known valve is that it can be closed only by exerting a pressure thereon, e.g. by means of a finger.

The object of the invention is to provide a solution to this problem. That is achieved in that said valve seat points towards the proximal end, such that the valve body and the valve seat can be made to interact with each other under the influence of an air flow from the proximal to the free end through the passage of the housing.

According to the invention, the valve body is a spherical segment, the concave side of which faces the valve seat. The exhaled air flows against the concave side of the spherical segment, thereby promoting closure of the valve. Another great advantage of the spherical shape is that the closure can occur virtually noiselessly, which is a very pleasant experience for the patient.

The valve body can be suspended under initial tension in many different ways. According to the invention, the spherical segment can have spring means which are distributed along the periphery and are supported on the housing, which spring means keep the spherical segment at rest in a position at a distance from the valve seat.

The spring means can comprise strips, each strip being curved, and near the end being accommodated in a recess in the inside wall of the housing.

The strips can also be interconnected by a supporting ring at their end facing away from the spherical segment.

The spherical segment, the strips and the supporting ring can be an integral unit made of synthetic material such as silicone rubber. Such a design is cheap and can easily be changed by the patient.

In addition, a valve body made in this way can be made in various degrees of stiffness. This provides a further possibility for adaptation to individual patients. The speech valve assembly can also be adjusted to varying needs of the patient, such as sport, exertion, rest etc.

An additional advantage of the speech valve assembly according to the invention relates to coughing of the patient. As already mentioned, in the case of known valves the valve or the skin seal can shoot off in such a case. However, the speech valve assembly according to the invention has the great advantage that the valve body can be reached directly from the outside through the fact that the filter does not block access to it. As a result of this, when the patient has a tickle in his throat he can press the valve body lightly with his finger, in a natural reflex, in such a way that the valve is prevented from closing and the air can escape without problems. This reflex is similar to putting one's hand to one's mouth during a coughing fit, and for that reason is therefore fully acceptable to the patient.

The housing bears an inward pointing valve seat at the free end. The valve seat is preferably adjustable in the direction of passage through the housing. That can be obtained, for example, by the valve seat forming part of a ring which can be screwed into the housing.

In order to facilitate cleaning or changing, the filter material can be accommodated loose in the housing. The filter material can be a disposable filter of corrugated paper material.

In a known manner, the housing can bear fixing means at the proximal end, for fixing said housing to a skin seal for fitting around the tracheal stoma.

The invention will be explained in greater detail below with reference to the exemplary embodiment shown in the figures.
Figure 1 shows a speech valve assembly according to the invention, as used in the case of a patient.
Figure 2 shows a longitudinal section through the speech valve assembly according to the invention.
Figure 3 shows a top view of the valve body.

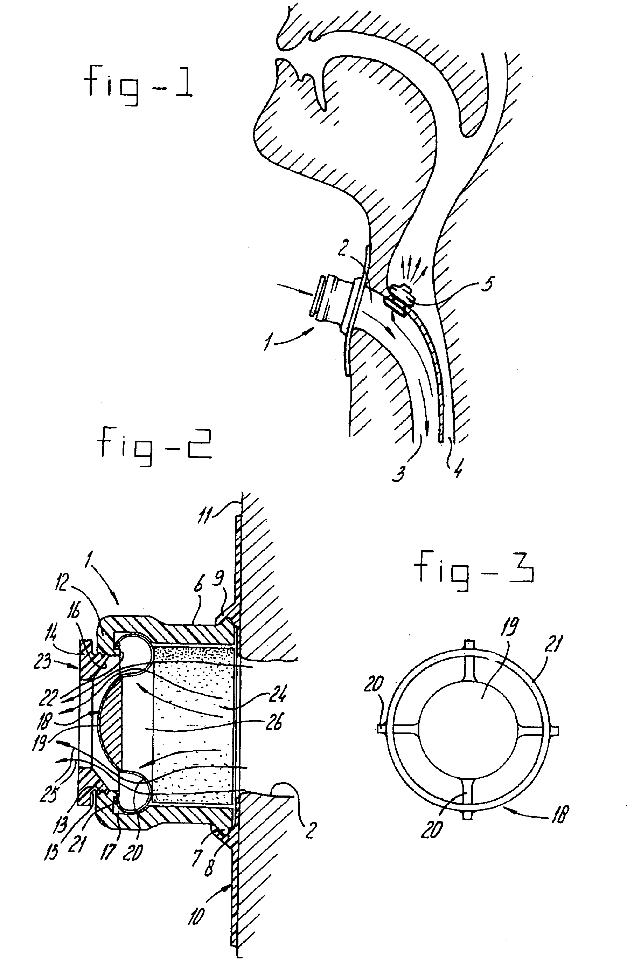

As shown in Figure 1, the speech valve assembly 1 according to the invention is fitted in the tracheal stoma 2 of a patient. Said tracheal stoma 2 forms the outlet of the trachea 3 of a laryngectomee, whose voice box has been removed.

A speech prosthesis, indicated in its entirety by 5, is fitted in the usual manner between the trachea 3 and the oesophagus 4. Said speech prosthesis 5 is known per se, and does not form part of the present invention.

If the patient wishes to speak, he must force air out of the trachea 3 through the speech prosthesis 5 and into the oesophagus 4. He can then develop speech through his oral cavity.

In order to be able to develop the excess pressure in the trachea 3 which is necessary for this purpose, the tracheal stoma 2 must be shut off, and the speech valve assembly 1 according to the invention has been provided for that purpose.

As shown in Figure 2, the speech valve assembly 1 according to the invention comprises a housing 6, which bears a peripheral rib 7 at its proximal end facing the tracheal stoma 2. Said peripheral rib 7 is clamped in or snapped into a correspondingly shaped groove 8 in the circular rib 9 of the skin seal indicated in its entirety by 10. Said skin seal is fixed in a known manner by means of an adhesive layer to the skin 11 of the patient, around the tracheal stoma 2.

At its end facing away from the tracheal stoma 2, the housing 6 has an inward projecting flange 12, which bears an internal screw thread 13. In addition, a ring 14 is provided and is screwed by means of corresponding screw thread 15 into the screw thread 13 of the flange 12.

The ring 14 determines the position of valve seat 16, which in the current exemplary embodiment is of a conically tapering design.

A recess 17 is provided in the inside wall of the housing 6, in which recess the valve, indicated in its entirety by 18, is supported. Said valve 18 is shown in top view in Figure 3.

The valve 18 is constructed from a spherical segment 19, four strips 20, and a ring 21.

Spherical segment 19, strips 20 and ring 21 form an integral unit made of synthetic material, for example silicone rubber.

As shown in Figure 2, the strips 20 are curved in such a way that they rest in the recess 17 of the housing 6. The ring 21 in this case rests against a downward pointing ridge 22 of the flange 12. By designing the strips 20 in the desired way, it is ensured that the spherical segment 19 is kept at a desired distance from the ring and thus from the seat 16. They can rest on the filter 24, to be described below.

Depending on the rigidity of the synthetic material used, the shape and thickness of the strips 20 and the distance between the spherical segment 19 and the seat 16, the valve remains in the open position during normal exhalation, as shown by the arrows 25.

During said exhalation, the filter, which is indicated in its entirety by 24, is heated and moistened. Said filter is likewise situated in the housing 6, and is confined between the valve body 18, in particular the strips 20 thereof, and the skin seal 10.

During inhalation, the cooler and drier ambient air is preheated and moistened by way of the filter 24, which is more pleasant for the patient. The great advantage of placing the filter 24 before the valve body 18, in the exhalation direction 25, is that the valve body 18 hardly becomes soiled with, for example, mucous. In addition, the filter 24 can easily be removed and then cleaned or thrown away. The patient need only remove the housing 6 of the skin seal 10, which is simple to perform by means of the snap connection 7, 8.

If the patient wishes to develop speech, the valve 23 must be closed. For this purpose, he generates a higher pressure or higher outflow velocity in his trachea 3, in such a way that the air trapped in the hollow space of the spherical segment 19 presses said spherical segment 19 against the seat 16.

The air can now no longer escape through the speech valve assembly 1 and chooses the route through the speech prosthesis 5.

During closure the valve 23 develops little noise, since the shapes of spherical segment 19 and valve seat 16, which are adapted to each other, do not generate any clicking noise.

If he wishes, the patient may replace the valve body 18 by a stiffer or more flexible valve body, adapted to his needs. It is also possible to use the valve assembly I without filter 24 in it. As an alternative, the filter 24 alone can also be used in the valve assembly 1, without the valve body 18.

If the patient has a tickle in his throat, he can easily hold down the spherical segment 19 with a finger, so that the valve 23 is prevented from closing. This means that the speech valve assembly 1 can be held reliably in place.

## Claims

1. Speech valve assembly (1) for connection to the tracheal stoma (2) of a laryngectomee, comprising a housing (6) with a passage (26) extending between a proximal end adapted to be positioned near the tracheal stoma (2) and a free end situated opposite, a filter means (24) situated near the proximal end, and a valve (23) situated on the side of the filter means (24) facing the free end, said valve (23) comprising a valve body (18) and a valve seat (16) which can be made to interact with each other against an initial tension, wherein the housing (6) bears said valve seat (14, 16) at the free end and said valve seat (16) points towards the proximal end, such that the valve body (18) and the valve seat (16) can be made to interact with each other under the influence of an air flow (25) from the proximal to the free end through the passage (26) of the housing (6), **characterized in that** the valve seat (14, 16) is adjustable in the direction of passage through the housing (6).

2. Speech valve assembly according to claim 1, in which the valve body (18) is a spherical segment (19), the convex side of which faces the valve seat (16).

3. Speech valve assembly according to claim 2, in which the spherical segment (19) has spring means (20) which are distributed along the periphery and are supported on the housing (6), which spring means (20) keep the spherical segment (19) at rest in a position at a distance from the valve seat (16).

4. Speech valve assembly according to claim 3, in which the spring means comprise strips (20).

5. Speech valve assembly according to claim 4, in which each strip (20) is curved, and near the end is accommodated in a recess (17) in the inside wall of the housing (6).

6. Speech valve assembly according to claim 4 or 5, in which the strips (20) are interconnected by a supporting ring (21) at their end facing away from the spherical segment (19).

7. Speech valve assembly according to claim 6, in which the spherical segment (19), the strips and the supporting ring (20) consist of an integral unit made of synthetic material.

8. Speech valve assembly according to claim 1 to 7, in which the valve seat (16) forms part of a ring (14) which can be screwed into the housing (6).

9. Speech valve assembly according to one of the preceding claims, in which the filter material (24) can be accommodated loose in the housing (6).

10. Speech valve assembly according to claim 9, in which the filter material (24) is a disposable filter of, preferably, foam, carbon and/or corrugated paper material.

11. Speech valve assembly according to one of the preceding claims, in which the housing (6) bears fixing means (7) at the proximal end, for fixing said housing to a skin seal (10) for fitting around the tracheal stoma.

## Patentansprüche

1. Sprachventilaufbau (1) für die Verbindung zu dem Stoma (2) einer Trachea bei einem Patienten, dessen Kehlkopf entfernt worden ist mit einem Gehäuse (6) mit einem Durchgang (26), der sich zwischen einem nahegelegenen Ende, welches dafür ausgelegt ist, in der Nähe des Stomas (2) der Trachea angeordnet zu werden, und einem entgegengesetzt liegenden freien Ende erstreckt, einer Filtereinrichtung (24), die in der Nähe des naheliegenden Endes angeordnet ist, und einem Ventil (23), welches auf der Seite der Filtereinrichtung (24) angeordnet ist, die dem freien Ende zugewandt ist, wobei das Ventil (23) einen Ventilkörper (18) und einen Ventilsitz (16) aufweist, die gegen eine anfängliche Vorspannung miteinander in Wechselwirkung treten können, wobei das Gehäuse (6) sich an dem Ventilsitz (14, 16) an dem freien Ende abstützt und der Ventilsitz (16) in Richtung des nahegelegenen Endes weist, so daß der Ventilkörper (18) und der Ventilsitz (16) durch die Wirkung eines Luftstroms (25) von dem nahegelegenen zu dem freien Ende zu dem Durchgang (26) des Gehäuses (6) in Wechselwirkung miteinander gebracht werden können, **dadurch gekennzeichnet, daß** der Ventilsitz (14, 16) in Richtung des Durchgangs durch das Gehäuse (6) einstellbar ist.

2. Sprechventilaufbau nach Anspruch 1, wobei der Ventilkörper (18) ein Kugelsegment (19) ist, dessen konvexe Seite dem Ventilsitz (16) zugewandt ist.

3. Sprechventilaufbau nach Anspruch 2, wobei das Kugelsegment (19) eine Federeinrichtung (20) hat, die entlang des Umfangs verteilt ist und sich an dem Gehäuse (6) abstützt, wobei diese Federeinrichtung (20) das kugelförmige Segment (19) unter einem Abstand von dem Ventilsitz (16) in einer Ruheposition hält.

4. Sprechventilaufbau nach Anspruch 3, wobei die Federeinrichtung Streifen (20) aufweist.

5. Sprechventilaufbau nach Anspruch 4, wobei jeder Streifen (20) gekrümmt ist und in der Nähe des Endes in einer Aussparung (17) in der Innenwand des Gehäuses (6) aufgenommen ist.

6. Sprechventilaufbau nach Anspruch 4 oder 5, bei welchem die Streifen (20) durch einen Haltering (21) an ihrem Ende, welches von dem Kugelsegment (19) abgewandt ist, miteinander verbunden sind.

7. Sprechventilaufbau nach Anspruch 6, wobei das kugelförmige Segment (19), die Streifen und der Haltering (20) aus einer aus einem künstlichen bzw. synthetischen Material hergestellten einstückigen Einheit bestehen.

8. Sprechventilaufbau nach einem der Ansprüche 1 bis 7, wobei der Ventilsitz (16) Teil eines Rings (14) bildet, der in das Gehäuse (6) eingeschraubt werden kann.

9. Sprechventilaufbau nach einem der vorstehenden Ansprüche, wobei das Filtermaterial (24) lose in dem Gehäuse (6) aufgenommen werden kann.

10. Sprechventilaufbau nach Anspruch 9, wobei das Filtermaterial (24) ein wegwerfbarer Filter vorzugsweise aus einem Schaum bzw. Kunststoffschaum, Kohle und/oder gewelltem Papiermaterial ist.

11. Sprechventilaufbau nach einem der vorstehenden Ansprüche, wobei das Gehäuse (6) eine Befestigungseinrichtung (7) an dem nahegelegenen Ende aufweist, um das Gehäuse an einer Hautabdichtung (10) für die Anpassung entlang des Umfangs des Stomas der Trachea zu tragen.

## Revendications

1. Ensemble (1) à valve de parole destiné à être connecté à la stomie trachéenne (2) d'un laryngectomisé, comprenant un boîtier (6) ayant un passage (26) qui s'étend entre une extrémité proximale destinée à être disposée près de la stomie trachéenne (2) et une extrémité libre ayant une position opposée, un dispositif à filtre (24) disposé près de l'extrémité proximale, et une valve (23) disposée du côté du dispositif à filtre (24) tourné vers l'extrémité libre, la valve (23) comprenant un corps de valve (18) et un siège de valve (16) qui peuvent être mis en interaction mutuelle malgré une tension initiale, dans lequel le boîtier (6) porte le siège de valve (14, 16) à l'extrémité libre et le siège de valve (16) est tourné vers l'extrémité proximale, si bien que le corps de valve (18) et le siège de valve (16) peuvent être mis en interaction mutuelle sous l'influence d'un courant d'air (25) allant de l'extrémité proximale vers l'extrémité libre par l'intermédiaire du passage (26) du boîtier (6), **caractérisé en ce que** le siège de valve (14, 16) est ajustable dans la direction du passage formé dans le boîtier (6).

2. Ensemble à valve de parole selon la revendication 1, dans lequel le corps de valve (18) est un segment sphérique (19) dont le côté convexe est tourné vers le siège de valve (16).

3. Ensemble à valve de parole selon la revendication 2, dans lequel le segment sphérique (19) possède un dispositif à ressort (20) qui est réparti le long de la périphérie et qui est supporté sur le boîtier (6), le dispositif à ressort (20) maintenant le segment sphérique (19) au repos en position distante du siège de valve (16).

4. Ensemble à valve de parole selon la revendication 3, dans lequel le dispositif à ressort comprend des bandes (20).

5. Ensemble à valve de parole selon la revendication 4, dans lequel chaque bande (20) est courbée et, près de l'extrémité, est logée dans une cavité (17) de la paroi interne du boîtier (6).

6. Ensemble à valve de parole selon la revendication 4 ou 5, dans lequel les bandes (20) sont interconnectées par un anneau de support (21) à leur extrémité tournée du côté opposé au segment sphérique (19).

7. Ensemble à valve de parole selon la revendication 6, dans lequel le segment sphérique (19), les bandes et l'anneau de support (20) sont formées d'une unité intégrée constituée d'un matériau de synthèse.

8. Ensemble à valve de parole selon la revendication 1 à 7, dans lequel le siège de valve (16) fait partie d'un anneau (14) qui peut être vissé dans le boîtier (6).

9. Ensemble à valve de parole selon l'une des revendications précédentes, dans lequel le matériau (24) de filtre peut être logé avec du jeu dans le boîtier (6).

10. Ensemble à valve de parole selon la revendication 9, dans lequel le matériau (24) du filtre est un filtre jetable constitué de préférence de mousse, de carbone et/ou d'un matériau de papier ondulé.

11. Ensemble à valve de parole selon l'une des revendications précédentes, dans lequel le boîtier (6) porte un dispositif de fixation (7) à son extrémité proximale pour la fixation du boîtier à un joint d'étanchéité (10) avec la peau destiné à être ajusté autour de la stomie trachéenne.
